# EUROPEAN PATENT SPECIFICATION

(11) **EP 4 106 615 B1**
(45) Date of publication and mention of the grant of the patent: **29.04.2026**
(21) Application number: 21710816.6
(22) Date of filing: 17.02.2021
(51) Int. Cl.: A61B 5/00, A61B 10/00, G01N 1/02, A61B 5/08, A61B 5/145, A61B 5/1495, G01N 1/22, G01N 33/497

(54) **DEVICE FOR COLLECTION OF VOLATILE COMPOUNDS FROM SKIN FOR NON-INVASIVE MEASUREMENT OF BLOOD-GLUCOSE VALUES**
VORRICHTUNG ZUM SAMMELN VON FLÜCHTIGEN VERBINDUNGEN AUS DER HAUT ZUR NICHTINVASIVEN MESSUNG VON BLUTGLUKOSEWERTEN
DISPOSITIF DE COLLECTE DE COMPOSÉS VOLATILES À PARTIR DE LA PEAU POUR UNE MESURE NON INVASIVE DES VALEURS DE GLUCOSE SANGUIN

(30) Priority: 18.02.2020 EP 20157876
(43) Date of publication of application: 28.12.2022
(73) Proprietor: F. Hoffmann-La Roche AG, 4070 Basel (CH); Roche Diabetes Care GmbH, 68305 Mannheim (DE)
(72) Inventor: FINK, Herbert, 68305 Mannheim (DE); LIST, Hans, 68305 Mannheim (DE); KACHHADIA, Alpeshkumar, 68305 Mannheim (DE)
(74) Representative: J A Kemp LLP
(86) International application number: PCT/US2021/018279
(87) International publication number: WO 2021/167918

(56) References cited:
- WO-A1-2016/072513
- JP-A- 2010 107 414
- US-A1- 2004 147 885
- US-A1- 2011 034 792

## Description

### BACKGROUND

This disclosure relates generally to measurement of volatile compounds emitted via transdermal diffusion, and more specifically to non-invasive techniques for such measurement.

Monitoring the concentration of glucose in the blood (glycemia) is important for diabetes management. A conventional blood glucose test is performed by piercing the skin to draw blood and then applying the blood to a chemically active disposable medium. While non-invasive techniques for blood glucose measurement have been proposed, current non-invasive approaches suffer from shortcomings that limit their utility.

An example of a non-invasive blood glucose monitoring technology is a diffusion cell device described in U.S. Patent 5,279,543A, entitled "Device for iontophoretic non-invasive sampling or delivery of substances". The diffusion cell device is described as performing electrically enhanced sampling of bioactive substances from skin or mucosal surfaces without mechanical penetration. The device utilizes a positive electrode, a negative electrode, and an electrically insulating material separating the electrodes, placed on the skin surface. Although providing a potentially non-invasive form of blood glucose level monitoring, the device requires the placement of and activation of electrodes directly on the surface of the skin to monitor changes of bioactive materials.

Another example of a non-invasive blood glucose monitoring technology is a skin surface sampling system described in U.S. Patent 10,143,447B2 entitled "Skin surface sampling system". This system utilizes an elongated collection tube with a sampling head positioned on one end in contact with the patient's skin. A liquid supply absorbs volatile organic compounds (VOC) and semi-volatile organic compounds (SVOC) from the surface of the skin and collects the mixed liquid in a sample collection device. The system is operated by positioning the sampling head on the skin surface and then flushing the liquid supply through a set of channel grooves in the sampling head that direct the mixed liquid to the collection tube. Although providing a potentially non-invasive form of blood glucose level monitoring, the system requires a liquid capture system.

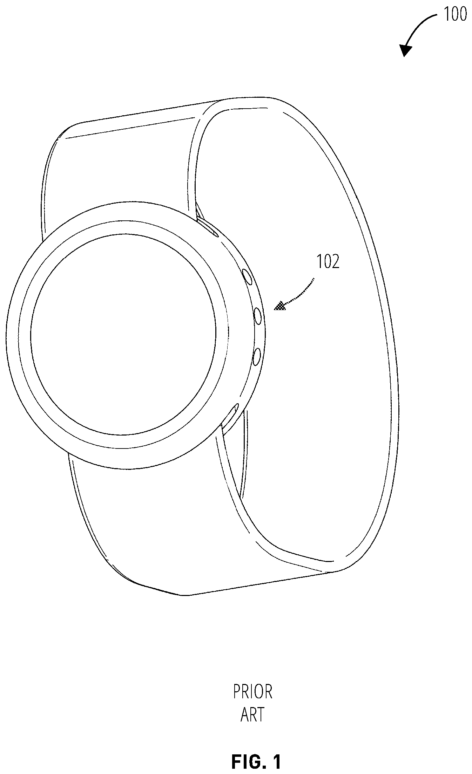

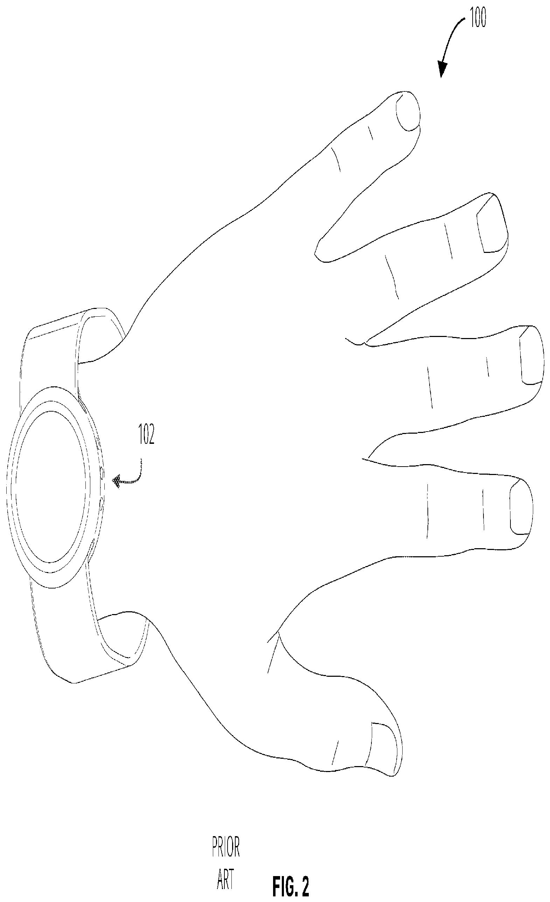

Another example of a potential non-invasive blood glucose monitoring technology is a breath analyzer 100 as depicted in FIG. 1 and FIG. 2. The breath analyzer 100 is a wrist mounted sampling device, similar to a smart watch, that includes a set of laterally positioned gas collection ports 102 intended to capture volatile organic compounds (VOC) and semi-volatile organic compounds (SVOC). The breath analyzer 100 utilizes an internal gas detector.

US 2004/0147885 Al discloses a skin permeable gas collector and skin permeable gas measuring apparatus. US 2011/0034792 Al discloses a noninvasive body chemistry monitor and method. WO 2016/072513 A1 discloses a method and device for measuring blood glucose level. JP 2010-107414 A discloses a method of collecting percutaneous gas, collecting device, and measurement method

### SUMMARY

In accordance with the present invention, there is disclosed a volumetric sampling apparatus as defined in claim 1.

The channel structure may form a gas flow path, such as a continuous curve between the inlet port and the outlet port. Such devices may be realized as wearables, such as smartwatches or fitness trackers or may be part of such wearables.

This disclosure further relates to a blood glucose level measurement method whereby a unitary body is mounted on a patient's skin to form a gas seal, the unitary body comprising a skin-facing surface in contact with the patient's skin and a top surface enclosing a structure, such as a channel structure open to the patient's skin at the skin-facing surface. A gas flow is then actuated through the structure between an inlet port and an outlet port of the unitary body.

This disclosure further relates to devices for volumetric sampling that include a unitary body with an inward spiral channel structure. The channel structure includes an inlet port and an outlet port and is open at a skin-facing surface of the unitary body.

### BRIEF DESCRIPTION OF THE DRAWINGS

To easily identify the discussion of any particular element or act, the most significant digit or digits in a reference number refer to the figure number in which that element is first introduced.
FIG. 1 depicts a prior art breath analyzer 100 in one aspect.
FIG. 2 depicts the prior art breath analyzer 100 in another aspect.
FIG. 3 depicts a patient mounted configuration 300 of a volumetric gas collector 400 in accordance with one embodiment.
FIG. 4 depicts a bottom isometric view of the volumetric gas collector 400 in accordance with one embodiment.
FIG. 5 depicts a bottom view of the volumetric gas collector 400.
FIG. 6 depicts a top isometric view of the volumetric gas collector 400.
FIG. 7 depicts a front view of the volumetric gas collector 400.
FIG. 8 depicts a rear view of the volumetric gas collector 400.
FIG. 9 depicts a lateral view of the volumetric gas collector 400.
FIG. 10 depicts the volumetric gas collector 400 incorporating a gas filter 1002 in accordance with one embodiment.
FIG. 11 depicts the volumetric gas collector 400 utilizing an inert gas supply 1104 in accordance with one embodiment.
FIG. 12 depicts the volumetric gas collector 400 utilizing a gradient heating element 1202 to facilitate gas flow in one embodiment in accordance with one embodiment.

### DESCRIPTION

Embodiments of an improved, non-invasive device are herein described for collection and measurement of volatile compounds emitted via transdermal diffusion from a patient's skin. "Volatile compounds" are molecules that are in gas phase at temperatures at or above approximately "room temperature", e.g., 20° Celsius. Such molecules have a vapor pressure at room temperature that is sufficient to generate a gas phase concentration (or partial pressure) in ambient air of at least 0.1 part per billion (ppb) by volume Non-limiting examples of volatile compounds are small molecules such as nitrogen oxide and carbon monoxide, larger molecules such as ethanol and benzene, and very large molecules such as proteins or other bio-molecules.

Embodiments of devices for volumetric sampling include a skin-facing surface with a structure that is open at the skin-facing surface. Such a structure in general allows the flow of a gas along one or more paths. The structure is not particularly limited as long as a gas flow through the structure is possible. The structure may be a channel structure forming a gas flow path, such as a continuous curve between the inlet port and the outlet port.

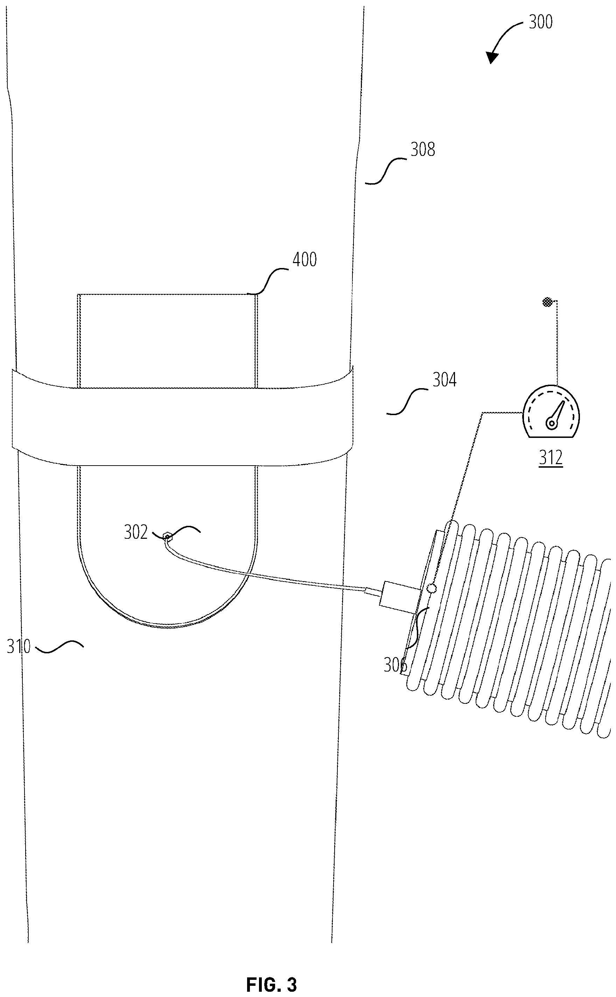

FIG. 3 depicts a patient-mounted configuration 300 of a volumetric gas collector 400 in accordance with one embodiment. The volumetric gas collector 400 may be mounted to the patient's arm at the inner elbow joint across a junction of the lower brachium region 308 and upper forearm region 310. A mounting strap 304 may be utilized to mount the volumetric gas collector 400 with sufficient force to form a substantially gas-tight seal between the volumetric gas collector 400 and the patient's skin. Volatile compounds emitted via transdermal diffusion from the portion of the patient's skin enclosed by the volumetric gas collector 400 are collected and migrated to the outlet port 302, e.g. under urging of a pressure source (not shown) coupled to and inducing negative pressure at the outlet port 302. From the outlet port 302 the volatile compounds migrate to a gas composition analyzer 306 such as a mass spectrometer. An ambient reference sensor 312 may optionally be utilized to provide a baseline reading of volatile compounds present in the environment. The baseline reading may be applied to adjust the reading obtained by the gas composition analyzer 306 for a more accurate assessment of the volatile compounds actually emitted via transdermal diffusion. The baseline reading may be taken once, or may be monitored continuously and averaged while the volumetric gas collector 400 is collecting gases.

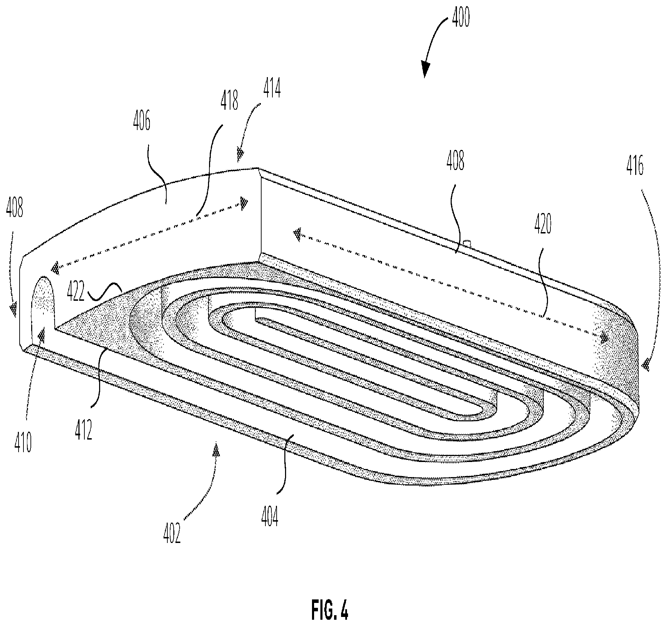

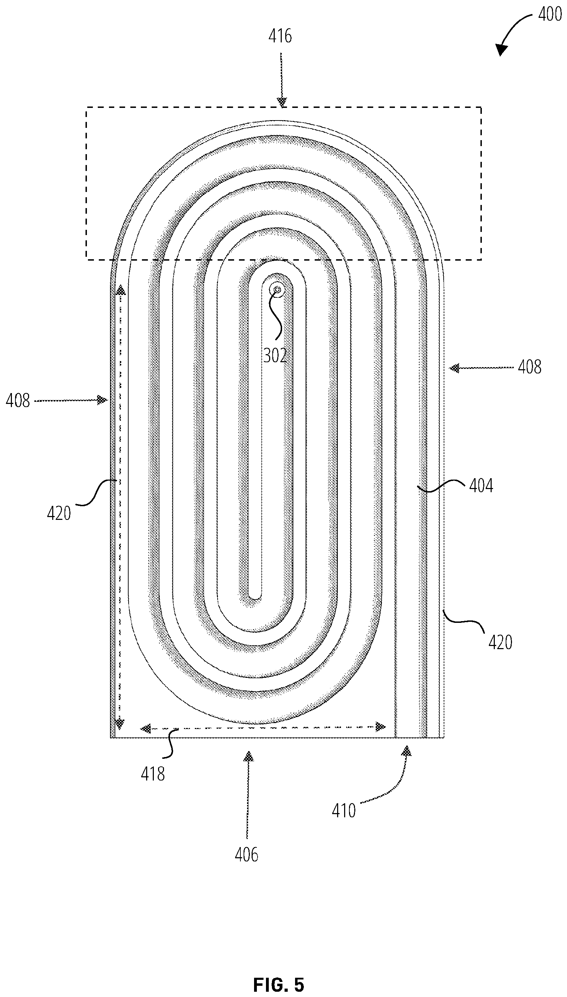

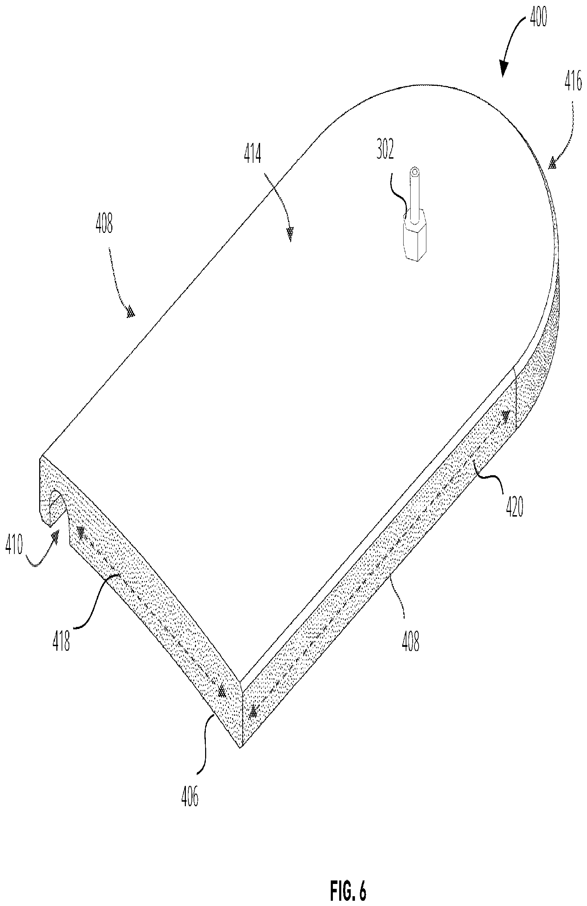

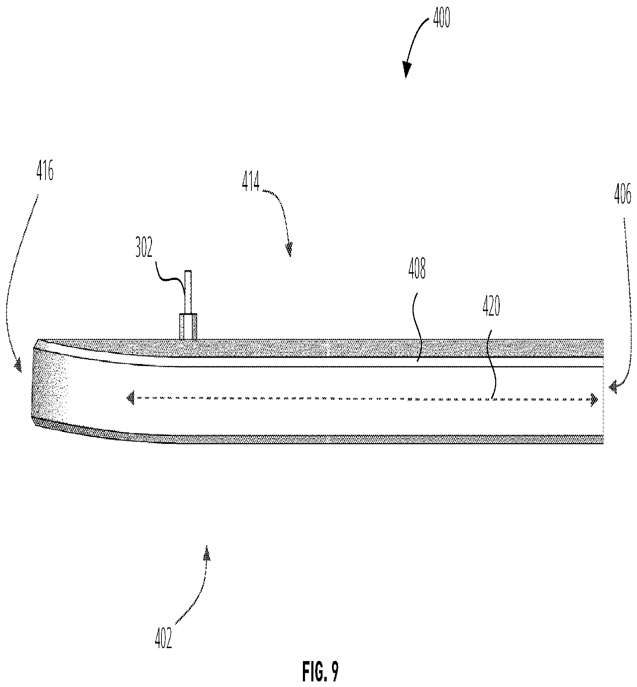

FIG. 4 - FIG. 9 depict a volumetric gas collector 400 in one embodiment. The volumetric gas collector 400 may be utilized to perform non-invasive blood glucose level measurement via the volumetric sampling of gases that are emitted via transdermal gas diffusion. Unlike prior art devices, the volumetric gas collector 400 does not utilize liquids or electrodes The volumetric gas collector 400 may be manufactured by milling a solitary unitary piece of material or manufactured using a 3D printer, for example, thus forming a unitary body. "Unitary body" refers to a single piece of material without joints or fasteners, into which the structure, such as a channel structure is formed. The volumetric gas collector 400 may for example be formed from noncorrosive materials such as Teflon, anodized titanium, plastic, or polymer materials that do not interact with or allow permeation by the volatile compounds to be collected, and which are not irritants to the skin of the intended patients.

The depicted embodiment has a form factor that is substantially rectangular on three sides of its periphery (the front surface 416 is substantially semi-circular). This and similar form factors may be advantageous for mounting on body parts that are more extensive in one dimension than in others. For example, a substantially rectangular (which herein includes rounded-rectangular - curved on both the front surface and back surface) form factor with a long axis 420 and short axis 418 may be advantageous in forming a larger sampling volume when mounted on the human arm, which is typically longer along the lower brachium region 308 to upper forearm region 3 10 direction (long axis) than it is wide (short axis, orthogonal to the radius and ulna). It will be appreciated that other form factors may capture this advantage, such as ovals or circular forms. In one embodiment where the intended mounted configuration 300 is on the human arm, the long axis 420 may be at least 1½ times a length of the short axis 418.

In the depicted embodiment the outlet port 302 is substantially centered along the short axis 418, but is off-center along the long axis 420. In form factors that exhibit a long axis and a short axis, such off-center placement of the outlet port 302 may advantageously provide a superior multivariate optimization of collection volume and gas flow rate in the channel structure 404.

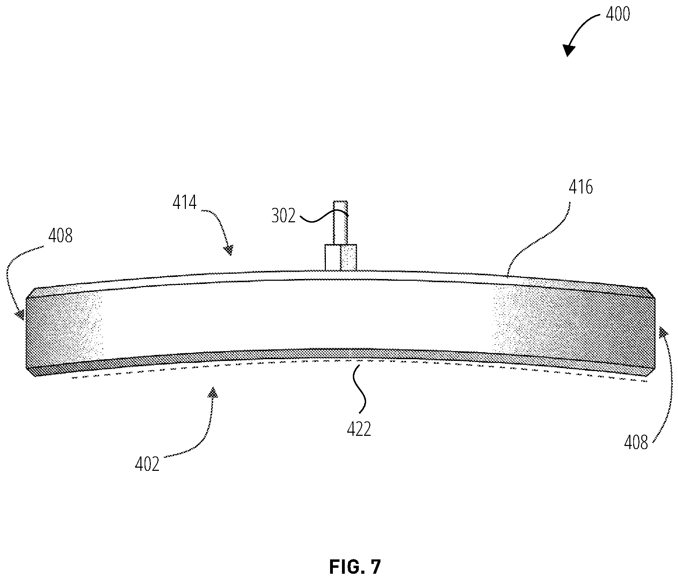

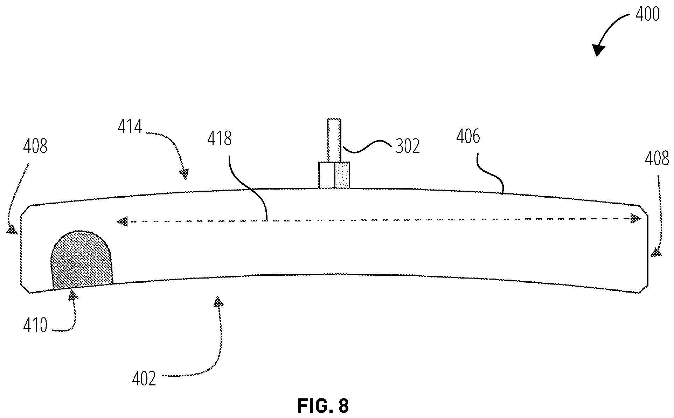

The depicted substantially rectangular form factor, which is merely one example of a possible form factor, distinguishes a back surface 406, front surface 416, and lateral surfaces 408, as well as a top surface 414 and skin-facing surface 402. Other form factors may only distinguish a subset of these surfaces. For example, oval or circular form factors may only distinguish a top surface and skin-facing surface. The unitary body may comprise a conformal curvature 422 of the skin-facing surface 402. The conformal curvature 422 (which extends across the short axis of the skin-facing surface 402, including the walls of the channel structure 404) may improve engagement with the curvatures of a patient's arm or body and thus form a gas vapor seal preventing or reducing escape of volatile compounds from the channel structure 404 to the environment, and preventing/reducing the gas flow from taking short cuts (multiple paths) through the channel structure 404. In one embodiment the conformal curvature 422 is concave, as depicted in FIG. 7 and FIG. 8.

Yet other form factors such as polygons with more than four sides may distinguish additional lateral surfaces. Some form factors may take the form of "shells" wherein the top surface is curved down to intersect the skin-facing surface, in which case there is no distinguished lateral surface. Regardless of the form factor, reference to the "periphery" of the volumetric gas collector device refers to the peripheral boundary between the skin-facing surface and the environment.

The channel structure 404 is depicted as doubling back along the long axis and spiraling inward from the periphery to the outlet port 302. However in other embodiments exhibiting a long axis and a short axis, the channel structure 404 may double back along the short axis 418.

The depicted channel structure 404 is a continuous curve. A "continuous curve" herein refers to an unbroken and unbranched path for gas flow from one point to another, in which changes in gas flow direction are effected without right or acute straight-line inner angles. A "straight-line" angle is one formed by the intersection of two straight sections having different directions. A continuous curve may include some straight (uncurved) sections, and may in some cases effect changes in gas flow direction by intersection straight sections at wide (e.g., greater than 100 degree) obtuse angles. Like smooth curves to change gas flow direction, such straight-line obtuse angles may be less prone than right or acute straight-line angles to creating dead space in the gas flow. The inward spiral channel structure in the depicted embodiments is an example of a continuous curve that includes some straight sections and some curved sections (where gas flow changes direction).

"Inward spiral" refers to a channel structure with an inlet port at or near the periphery, that spirals inward toward a center axis of device where the outlet port is located. With inward spirals, one end of the channel structure can form an opening for the inlet port at the periphery while the other end of the channel structure terminates internally within the enclosure of the skin-facing surface, and the outlet port traverses from the channel structure through the top surface (e.g., via a hole drilled from the top surface to the channel structure). In some embodiments, the inward spiral channel structure may be fully contained within the skin-facing surface (the channel structure does not terminate at an opening on the periphery), in which case the inlet port would, like the outlet port, need to traverse up through the top surface (e.g., via a hole drilled from the top surface to the channel structure).

An inward spiral channel structure comprises at least one endpoint that is interior to the enclosure of the skin-facing surface 402, and therefore the outlet port 302 necessarily traverses from the channel structure 404 to the top surface 414 for such channel structures. The outlet port 302 may in these cases comprise a fitting to enable a negative pressure source, such as a pump or vacuum, to be applied at the outlet port 302.

In some embodiments, such as circular form factors, there may be no distinguished axis along which the channel structure 404 doubles back, and the channel structure 404 may spiral inward from the periphery to the outlet port 302.

It should therefore be appreciated that the volumetric gas collector 400 may be embodied in a variety of form factors and channel structure 404 configurations, depending on the parameters of the intended application. It should also be appreciated that in some embodiments the gas flow may be implemented in the reverse direction than depicted. For example, the locations of the inlet port 410 and the outlet port 302 may be interchanged such that pressure is applied from at or near an interior point and volatile compounds are collected for analysis/measurement at a location near or on the periphery.

The channel structure 404 is formed into the skin-facing surface 402, forming a skin-contact surface 412 along the walls of the channel structure 404. The skin-contact surface 412 supports mounting (e.g., via a mounting strap 304) the volumetric gas collector 400 non-invasively on the surface of the skin of patients. Volatile compounds emitted from the skin via transdermal gas diffusion collect in the channel structure 404 and flow toward the outlet port 302. The channel structure 404 may form a gas-impermeable barrier to the volatile compounds to prevent or reduce escape to and/or contamination from the environment, and to prevent or reduce short cuts by the gas flow when traversing the channel structure 404.

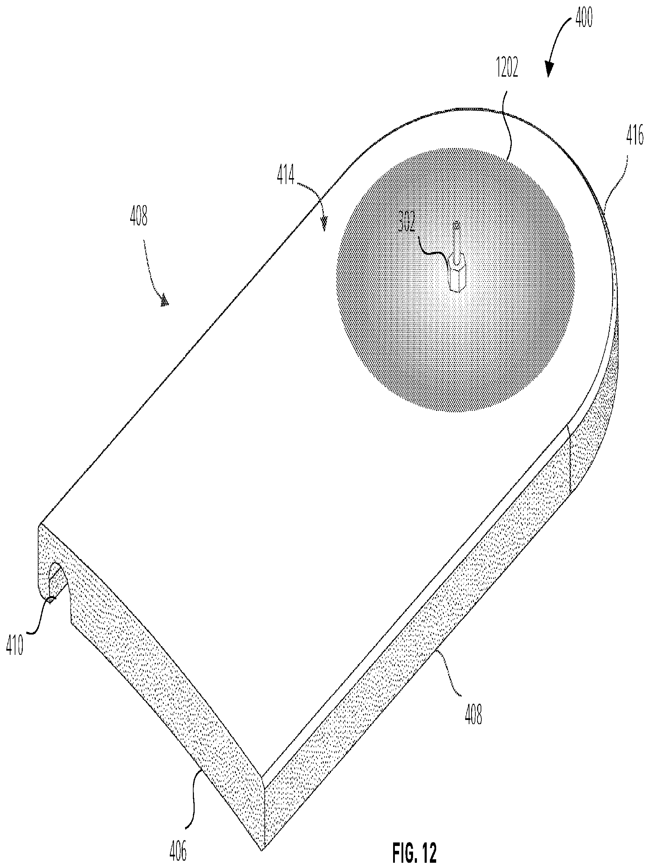

The gas flow induced in the channel structure 404 may be partially thermodynamic (tending toward regions of lower volatile compound density and/or temperature) and may be assisted by an external force such as a pump (e.g., providing negative pressure at the outlet port 302 or positive pressure at the inlet port 410), pressurized gas source (e.g., at the inlet port 410), or vacuum (e.g., at the outlet port 302). A negative pressure is generally preferable to a positive pressure due to the inhibitive effect that a positive pressure may have on transdermal diffusion of volatile compounds from the skin. The gas flow rate may also be enhanced thermodynamically using, for example, a heating element (see FIG. 12). In a mounted configuration 300, a gas composition analyzer 306 may be coupled to the outlet port 302 to characterize the particles of volatile compounds and to determine, for example, blood glucose levels. The gas composition analyzer 306 may be preferably a mass spectrometer or other gas sensors used to continuously analyze VOC-components from ambient air, such as metal oxide sensors (MOS), infrared sensors, electrochemical sensors or sensors with polymeric coating, or other sensors.

In some embodiments negative pressure is used at the outlet port 302. Negative pressure is generated by a suction process. Suction is applied intermittently, with a relatively high pressure difference, so that sufficiently high flow velocity is generated, which results in a Reynolds Number of over 10000. The gas flow within the structure then is turbulent instead of laminar. It is well known to a person skilled in the art, that the transition from a laminar to a turbulent gas flow in a pipe usually occurs already at a Reynolds Number over 2300. The high pressure difference resulting a high Reynolds Number over 10000 in this embodiment is necessary for a turbulent flow since the gas flow can be considerably slowed down within parts of the structure. A turbulent flow has the advantage of reaching potential niches, corners, and other dead space of the structure.

The channel structure 404 includes an inlet port 410 that replaces gases drawn from the outlet port 302 for analysis. The inlet port 410 may be open to ambient air, or may couple to a pressurized or unpressurized source of replacement gas, such as an inert gas. "Ambient air" refers to the external atmosphere surrounding a patient and the volatile compounds sampling device. An unpressurized source of inert gas may be preferred due to the inhibitive effects of positive pressure inside the channel structure 404 on the transdermal diffusion of volatile compounds from the skin.

In some embodiments, for example as depicted, the channel structure 404 may be formed to have a consistent width from the inlet port 410 to the outlet port 302. By maintaining a consistent volume along the length of the channel structure 404, a constant gas flow rate towards the outlet port 302 from the inlet port 410 may be facilitated. The curvature and dimensions (e.g., width and depth) of the channel structure 404 may be selected for example based on a multivariate optimization of collection volume and gas flow rate for a particular form factor of the device. Constraints on the width of the channel structure 404 include lower limits on narrowness to prevent constriction of gas flow, and lower limits on width of the walls of the channel structure 404 to prevent leakage of volatile compounds through the walls. The depth of the channel structure 404 may also be constrained to a lower limit to accommodate bulging of a patient's skin into the channel structure 404, without creating inhibition to gas flow in the mounted configuration 300.

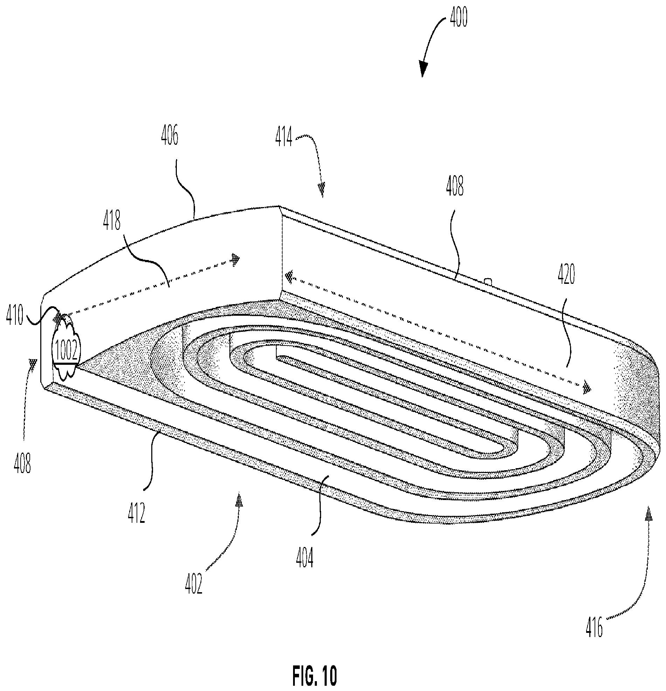

FIG. 10 depicts a volumetric gas collector 400 with a gas filter 1002 positioned in or proximal to the inlet port 410. The gas filter 1002 may be provided to reduce volatile compounds contaminants from entering the channel structure 404 by way of the inlet port 410.

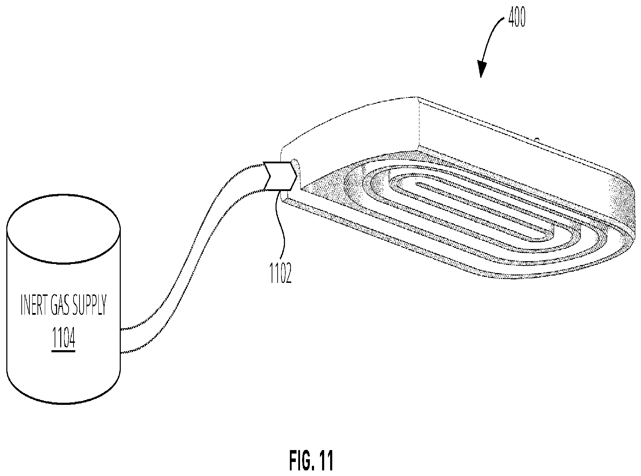

FIG. 11 depicts a volumetric gas collector 400 operatively coupled to an inert gas supply 1104 through a coupling 1102 connected to the inlet port 410. The inert gas supply 1104 may be provided as a way to control the introduction of unwanted molecules that may be pulled into the channel structure 404 through the inlet port 410. The inert gas supply 1104 may be a gas such as nitrogen that has a low reactivity with the volatile compounds to be collected. The inert gas supply 1104 may in some cases be pressurized to facilitate the gas flow rate in the channel structure 404, however any pressure applied should be selected so as not to inhibit transdermal diffusion of the volatile compounds from the patient's skin.

FIG. 12 depicts a volumetric gas collector 400 with a gradient heating element 1202. The gradient heating element 1202 may be configured to generate a temperature gradient from the lateral surface 408 toward the outlet port 302. More generally, the gradient heating element 1202 may generate a temperature gradient that decreases from the inlet port 410 toward the outlet port 302, overall, thus facilitating gas flow via thermodynamics. In one embodiment the gradient heating element 1202 may be implemented as light-emitting diodes (LEDs) in the channel structure 404, producing not only heat but potentially increasing the transdermal diffusion of volatile compounds from the patient's skin via optical stimulation. The gradient heating element 1202 may thus improve gas flow and/or the concentration of volatile compounds in the channel structure 404, and may be a separate component (e.g., heating pad) from the unitary body or integral with it (e.g., an integral heating element or LEDs). In some embodiments, the heating element or elements are used to generate heat but not necessarily a thermal gradient In this embodiment the heating does not necessarily facilitate gas flow thermodynamically, but is nonetheless advantageous for stimulating the transdermal diffusion of volatile compounds from the skin.

Within this disclosure, different entities may be described or claimed as "configured" to perform one or more tasks or operations. This formulation-[entity] configured to [perform one or more tasks]-is used herein to refer to structure (i.e., something physical). More specifically, this formulation is used to indicate that such a physical structure is arranged to perform the one or more tasks during operation. A physical structure can be said to be "configured to" perform some task even if the physical structure is not currently being operated. The term "configured to" is not intended to mean "configurable to."

As used herein, the term "based on" is used to describe one or more factors that affect a determination. This term does not foreclose the possibility that additional factors may affect the determination. That is, a determination may be solely based on specified factors or based on the specified factors as well as other, unspecified factors. Consider the phrase "determine A based on 13." This phrase specifies that B is a factor that is used to determine A or that affects the determination of A. This phrase does not foreclose that the determination of A may also be based on some other factor, such as C. This phrase is also intended to cover an embodiment in which A is determined based solely on B. As used herein, the phrase "based on" is synonymous with the phrase "based at least in part on."

As used herein, the phrase "in response to" describes one or more factors that trigger an effect. This phrase does not foreclose the possibility that additional factors may affect or otherwise trigger the effect. That is, an effect may be solely in response to those factors, or may be in response to the specified factors as well as other, unspecified factors. Consider the phrase "perform A in response to B." This phrase specifies that B is a factor that triggers the performance of A. This phrase does not foreclose that performing A may also be in response to some other factor, such as C. This phrase is also intended to cover an embodiment in which A is performed solely in response to B.

As used herein, the terms "first," "second," etc. are used as labels for nouns that they precede, and do not imply any type of ordering (e.g., spatial, temporal, logical, etc.), unless stated otherwi se.

When used in the claims, the term "or" is used as an inclusive or and not as an exclusive or. For example, the phrase "at least one of x, y, or z" means any one of x, y, and z, as well as any combination thereof.

## Claims

1. A volumetric sampling apparatus (400) comprising:
a unitary body comprising an inwardly spiraling channel structure (404), wherein the channel structure comprises an inlet port (410) and an outlet port (302), and wherein the channel structure is open at a skin-facing surface (402) of the unitary body and is enclosed by a top surface (414) of the unitary body; and
a heating element (1202) configured to generate a temperature gradient along the channel structure between the inlet port and the outlet port;
wherein the inlet port is open to ambient air; and wherein the outlet port is configured for coupling to a negative pressure source.

2. The volumetric sampling apparatus of claim **1,** wherein the outlet port is further configured for coupling to a gas composition analyzer (306).

3. The volumetric sampling apparatus of claim **1,** further comprising a gas filter (1002) positioned in or proximal to the inlet port, the gas filter being configured to reduce volatile compounds contaminants from entering the channel structure by way of the inlet port when the channel structure is positioned on a patient's skin.

4. The volumetric sampling apparatus of any one of claims 1 to 3, wherein the channel structure forms a continuous curve between the inlet port and the outlet port.

5. The volumetric sampling apparatus of any one of claims 1 to 4, wherein the skin-facing surface comprises a conformal curvature configured for a sealing-contact with a patient's skin.

6. The volumetric sampling apparatus of claim 5, wherein the conformal curvature is concave.

7. The volumetric sampling apparatus of any one of claims 1 to 6, further comprising a heating element (1202) configured to stimulate transdermal diffusion of volatile compounds from a patient's skin.

8. The volumetric sampling apparatus of any one of claims 1 to 7, wherein the temperature gradient decreases from the inlet port toward the outlet port.

9. The volumetric sampling apparatus according to any one of claims 1 to 8, wherein the channel structure has a consistent width from the inlet port to the outlet port.

10. The volumetric sampling apparatus according to any one of claims 1 to 9, wherein the volumetric sampling apparatus is configured for operatively coupling to an inert gas supply (1104) through a coupling (1102) connected to the inlet port.

11. A blood glucose level measurement method comprising:
mounting the volumetric sampling apparatus (400) of any one of claims 1 to 10 on a patient's skin to form a gas seal, the skin-facing surface being in contact with the patient's skin;
actuating a gas flow through the structure between the inlet port and the outlet port of the unitary body by applying negative pressure at the outlet port to draw ambient air from the inlet port through the channel structure; and
measuring volatile compounds in gases collected at the outlet port using a gas composition analyzer (306).

12. The blood glucose level measurement method of claim 11, further comprising applying thermal heating to the unitary body.

13. The blood glucose level measurement method of claim 11 or 12, further comprising:
measuring a baseline level of the volatile compounds in ambient air; and
applying the baseline level to adjust a measure of the volatile compounds in the gases collected at the outlet port.

## Patentansprüche

1. Volumetrische Probenentnahmevorrichtung (400), umfassend:
einen einstückigen Körper, der eine sich spiralförmig nach innen windende Kanalstruktur (404) umfasst, wobei die Kanalstruktur eine Einlassöffnung (410) und eine Auslassöffnung (302) umfasst und wobei die Kanalstruktur an einer der Haut zugewandten Oberfläche (402) des einstückigen Körpers offen ist und von einer oberen Oberfläche (414) des einstückigen Körpers umschlossen ist; und
ein Heizelement (1202), das konfiguriert ist, um einen Temperaturgradienten entlang der Kanalstruktur zwischen der Einlassöffnung und der Auslassöffnung zu erzeugen;
wobei die Einlassöffnung zur Umgebungsluft offen ist; und wobei die Auslassöffnung zum Koppeln an eine Unterdruckquelle konfiguriert ist.

2. Volumetrische Probenentnahmevorrichtung nach Anspruch 1, wobei die Auslassöffnung ferner zum Koppeln an einen Gaszusammensetzungsanalysator (306) konfiguriert ist.

3. Volumetrische Probenentnahmevorrichtung nach Anspruch 1, ferner umfassend einen Gasfilter (1002), der in oder nahe der Einlassöffnung positioniert ist, wobei der Gasfilter konfiguriert ist, um das Eindringen flüchtiger kontaminierender Verbindungen über die Einlassöffnung in die Kanalstruktur zu reduzieren, wenn die Kanalstruktur auf der Haut eines Patienten positioniert ist.

4. Volumetrische Probenentnahmevorrichtung nach einem der Ansprüche 1 bis 3, wobei die Kanalstruktur eine kontinuierliche Kurve zwischen der Einlassöffnung und der Auslassöffnung bildet.

5. Volumetrische Probenentnahmevorrichtung nach einem der Ansprüche 1 bis 4, wobei die der Haut zugewandte Oberfläche eine konforme Krümmung umfasst, die für einen Dichtungskontakt mit der Haut eines Patienten konfiguriert ist.

6. Volumetrische Probenentnahmevorrichtung nach Anspruch 5, wobei die konforme Krümmung konkav ist.

7. Volumetrische Probenentnahmevorrichtung nach einem der Ansprüche 1 bis 6, ferner umfassend ein Heizelement (1202), das konfiguriert ist, um die transdermale Diffusion von flüchtigen Verbindungen aus der Haut eines Patienten zu stimulieren.

8. Volumetrische Probenentnahmevorrichtung nach einem der Ansprüche 1 bis 7, wobei der Temperaturgradient von der Einlassöffnung zur Auslassöffnung abnimmt.

9. Volumetrische Probenentnahmevorrichtung nach einem der Ansprüche 1 bis 8, wobei die Kanalstruktur eine konstante Breite von der Einlassöffnung zur Auslassöffnung aufweist.

10. Volumetrische Probenentnahmevorrichtung nach einem der Ansprüche 1 bis 9, wobei die volumetrische Probenentnahmevorrichtung zur operativen Kopplung an eine Inertgasversorgung (1104) durch eine Kopplung (1102), die mit der Einlassöffnung verbunden ist, konfiguriert ist.

11. Verfahren zur Messung von Blutglukosespiegeln, umfassend:
Anbringen der volumetrischen Probenentnahmevorrichtung (400) nach einem der Ansprüche 1 bis 10 auf der Haut eines Patienten, um eine Gasdichtung zu bilden, wobei die der Haut zugewandte Oberfläche in Kontakt mit der Haut des Patienten steht;
Führen eines Gasstroms durch die Struktur zwischen der Einlassöffnung und der Auslassöffnung des einstückigen Körpers durch Anlegen von Unterdruck an der Auslassöffnung, um Umgebungsluft von der Einlassöffnung durch die Kanalstruktur zu ziehen; und
Messen flüchtiger Verbindungen in Gasen, die an der Auslassöffnung gesammelt wurden, unter Verwendung eines Gaszusammensetzungsanalysators (306).

12. Verfahren zur Messung von Blutglukosespiegeln nach Anspruch 11, ferner umfassend das Anwenden von thermischer Erwärmung auf den einstückigen Körper.

13. Verfahren zur Messung von Blutglukosespiegeln nach Anspruch 11 oder 12, ferner umfassend:
Messen eines Ausgangsspiegels der flüchtigen Verbindungen in der Umgebungsluft; und
Anwenden des Ausgangsspiegels, um ein Maß der flüchtigen Verbindungen in den Gasen, die an der Auslassöffnung gesammelt wurden, anzupassen.

## Revendications

1. Appareil d'échantillonnage volumétrique (400) comprenant :
un corps unitaire comprenant une structure de canal en spirale vers l'intérieur (404), dans lequel la structure de canal comprend un orifice d'entrée (410) et un orifice de sortie (302), et dans lequel la structure de canal est ouverte au niveau d'une surface faisant face à la peau (402) du corps unitaire et est entourée par une surface supérieure (414) du corps unitaire ; et
un élément chauffant (1202) configuré pour générer un gradient de température le long de la structure de canal entre l'orifice d'entrée et l'orifice de sortie ;
dans lequel l'orifice d'entrée est ouvert à l'air ambiant ; et dans lequel l'orifice de sortie est configuré pour être accouplé à une source de pression négative.

2. Appareil d'échantillonnage volumétrique selon la revendication 1, dans lequel l'orifice de sortie est en outre configuré pour être accouplé à un analyseur de composition de gaz (306).

3. Appareil d'échantillonnage volumétrique selon la revendication 1, comprenant en outre un filtre à gaz (1002) positionné dans ou à proximité de l'orifice d'entrée, le filtre à gaz étant configuré pour réduire l'entrée des composés contaminants volatils dans la structure de canal par le biais de l'orifice d'entrée lorsque la structure de canal est positionnée sur la peau d'un patient.

4. Appareil d'échantillonnage volumétrique selon l'une quelconque des revendications 1 à 3, dans lequel la structure de canal forme une courbe continue entre l'orifice d'entrée et l'orifice de sortie.

5. Appareil d'échantillonnage volumétrique selon l'une quelconque des revendications 1 à 4, dans lequel la surface faisant face à la peau comprend une courbure conforme configurée pour un contact étanche avec la peau d'un patient.

6. Appareil d'échantillonnage volumétrique selon la revendication 5, dans lequel la courbure conforme est concave.

7. Appareil d'échantillonnage volumétrique selon l'une quelconque des revendications 1 à 6, comprenant en outre un élément chauffant (1202) configuré pour stimuler la diffusion transdermique de composés volatils à partir de la peau d'un patient.

8. Appareil d'échantillonnage volumétrique selon l'une quelconque des revendications 1 à 7, dans lequel le gradient de température diminue de l'orifice d'entrée vers l'orifice de sortie.

9. Appareil d'échantillonnage volumétrique selon l'une quelconque des revendications 1 à 8, dans lequel la structure de canal a une largeur constante de l'orifice d'entrée jusqu'à l'orifice de sortie.

10. Appareil d'échantillonnage volumétrique selon l'une quelconque des revendications 1 à 9, dans lequel l'appareil d'échantillonnage volumétrique est configuré pour être accouplé fonctionnellement à une alimentation en gaz inerte (1104) par l'intermédiaire d'un raccord (1102) relié à l'orifice d'entrée.

11. Procédé de mesure du taux de glucose sanguin comprenant :
le montage de l'appareil d'échantillonnage volumétrique (400) selon l'une quelconque des revendications 1 à 10 sur la peau d'un patient pour former une étanchéité aux gaz, la surface faisant face à la peau étant en contact avec la peau du patient ;
l'actionnement d'un écoulement de gaz à travers la structure entre l'orifice d'entrée et l'orifice de sortie du corps unitaire par application d'une pression négative au niveau de l'orifice de sortie pour aspirer l'air ambiant à partir de l'orifice d'entrée à travers la structure de canal ; et
la mesure de composés volatils dans les gaz collectés au niveau de l'orifice de sortie à l'aide d'un analyseur de composition de gaz (306).

12. Procédé de mesure du taux de glucose sanguin selon la revendication 11, comprenant en outre l'application d'un chauffage thermique sur le corps unitaire.

13. Procédé de mesure du taux de glucose sanguin selon la revendication 11 ou 12, comprenant en outre :
la mesure d'un taux de référence des composés volatils dans l'air ambiant ; et
l'application du taux de référence pour ajuster une mesure des composés volatils dans les gaz collectés au niveau de l'orifice de sortie.
